Europäisches Patentamt

European Patent Office

Office européen des brevets

(19)

(11) **EP 0 589 981 B1**

(12) **EUROPÄISCHE PATENTSCHRIFT**

(45) Veröffentlichungstag und Bekanntmachung des Hinweises auf die Patenterteilung:
**23.10.1996 Patentblatt 1996/43**

(51) Int. Cl.$^6$: **A61K 9/20**, A61K 9/24, A61K 9/28, A61K 9/32, A61K 9/54, A61K 31/44

(21) Anmeldenummer: **92912364.4**

(22) Anmeldetag: **13.06.1992**

(86) Internationale Anmeldenummer:
**PCT/EP92/01341**

(87) Internationale Veröffentlichungsnummer:
**WO 92/22284 (23.12.1992 Gazette 1992/32)**

(54) **PANTOPRAZOL ENTHALTENDE ORALE DARREICHUNGSFORMEN**

ORAL-ADMINISTRATION FORMS OF A MEDICAMENT CONTAINING PANTOPRAZOL

FORMES DE PRESENTATION D'UN MEDICAMENT CONTENANT DU PANTOPRAZOL ET ADMINISTRE PAR VOIE ORALE

(84) Benannte Vertragsstaaten:
**AT BE CH DE DK ES FR GB GR IT LI LU MC NL PT SE**

(30) Priorität: **17.06.1991 CH 1788/91**

(43) Veröffentlichungstag der Anmeldung:
**06.04.1994 Patentblatt 1994/14**

(73) Patentinhaber: **BYK GULDEN LOMBERG CHEMISCHE FABRIK GMBH
D-78403 Konstanz (DE)**

(72) Erfinder:
• **DIETRICH, Rango
D-7750 Konstanz 16 (DE)**
• **NEY, Hartmut
D-7750 Konstanz (DE)**

(56) Entgegenhaltungen:
**EP-A- 0 244 380          EP-A- 0 247 983
EP-A- 0 342 522**

Bemerkungen:
Verbunden mit 92110021.0/0519365 (europäische Anmeldenummer/Veröffentlichungsnummer) durch Entscheidung vom 25.10.94.

EP 0 589 981 B1

**Beschreibung**

<u>Stand der Technik</u>

In der europäischen Patentanmel dung EP-A-244 380 werden orale Darreichungsformen für säurelabile Wirkstoffe aus der Klasse der $H^+/K^+$-ATPase-Hemmer mit Pyridylmethylsulfinyl-1H-benzimidazol-Struktur beschrieben, die einen Kern, eine Zwischenschicht und eine magensaftresistente äußere Schicht aufweisen. In der europäischen Patentanmeldung EP-A-247 983 werden die in der EP-A-244 380 offenbarten Formulierungen im Zusammenhang mit dem $H^+/K^+$-ATPase-Hemmer Omeprazol beschrieben und beansprucht.

Bei den in den europäischen Patentanmeldungen EP-A-244 380 und EP-A-247 983 beanspruchten Darreichungsformen wird für die säurelabilen Wirkstoffe eine Stabilisierung insbesondere durch den Zusatz von Basen zum Kern und somit eine Erhöhung des pH-Wertes erreicht; für die Erzielung einer ausreichenden Lagerstabilität müssen jedoch sowohl bei der Herstellung als auch bei der Lagerung bestimmte Bedingungen eingehalten werden, die mit einer optimalen galenischen Formulierung und einer problemlosen Vorratshaltung nur schlecht in Einklang zu bringen sind. So heißt es in der EP-A-247 983 sinngemäß: "Für die Langzeitstabilität bei der Lagerung ist es wesentlich, daß der Wassergehalt der den Wirkstoff Omeprazol enthaltenden Darreichungsform (magensaftresistent überzogene Tabletten, Kapseln und Pellets) niedrig gehalten wird und bevorzugt nicht mehr als 1,5 Gew.-% beträgt. Demzufolge sind Endverpackungen mit in Hartgelatinekapseln abgefüllten, magensaftresistent überzogenen Pellets bevorzugt mit Trockenmitteln zu versehen, die den Wassergehalt der Gelatinehüllen so weit senken, daß der Wassergehalt in den Pellets 1,5 Gew.-% nicht überschreitet".

Der bei der Herstellung von Pelletkernen aus Stabilitätsgründen niedrig zu haltende Wassergehalt bewirkt nun, daß die für die Pelletkernherstellung zu extrudierende Masse nicht ausreichend plastisch ist, um das Extrudat anschließend zu sphärischen Partikeln runden zu können. Es entstehen vielmehr zylindrische Körper, die bei den anschließenden Coating-Schritten an den Enden weniger dicke Lackschichten erhalten und somit an diesen Stellen nicht die geforderte Magensaftresistenz aufweisen und überdies den Kern nicht sicher von der magensaftresistenten Schicht durch ein Sub-coating schützen, was für die Stabilität wesentlich ist.

Die aufgezeigten Stabilitätsprobleme treten auch auf, wenn man versucht, den $H^+/K^+$-ATPase-Hemmer Pantoprazol (prop. INN für die Verbindung 5-(Difluormethoxy)-2-[(3,4-dimethoxy-2-pyridyl)methylsulfinyl]-1H-benzimidazol) so zu formulieren, wie dies in den europäischen Patentanmeldungen EP-A-244 380 und EP-A-247 983 beschrieben ist.

<u>Beschreibung der Erfindung</u>

Überraschenderweise wurde nun gefunden, daß beim Verzicht auf bestimmte, als Tablettenhilfsstoffe häufig verwendete Füllstoffe und Bindemittel, wie sie für die Herstellung der Pellet- bzw. Tablettenkerne in den europäischen Patentanmeldungen EP-A-244 380 und EP-A-247 983 angegeben sind, die geschilderten Stabilitätsprobleme nicht auftreten. Diese Füllstoffe bzw. Bindemittel sind insbesondere Lactose, mikrokristalline Zellulose und Hydroxypropylzellulose.

Gegenstand der Erfindung ist somit ein den Wirkstoff Pantoprazol enthaltendes, oral zu applizierendes, magensaftresistentes Arzneimittel in Pellet- oder Tablettenform, das aus einem basisch reagierenden Pellet- oder Tablettenkern, einer oder mehreren inerten, wasserlöslichen Zwischenschicht(en) und einer magensaftresistenten äußeren Schicht besteht, und das dadurch gekennzeichnet ist, daß der Kern neben Pantoprazol bzw. neben einem Pantoprazol-Salz als Bindemittel Polyvinvlpyrrolidon und/oder Hydroxypropylmethylcellulose und gewünschtenfalls zusätzlich als inerten Füllstoff Mannit enthält.

Für eine basische Reaktion des Pellet- oder Tablettenkernes wird diesem - sofern die gewünschte Erhöhung des pH-Wertes nicht bereits durch Verwendung des Wirkstoff-Salzes erzielt wird - eine anorganische Base beigemischt. Hier seien beispielsweise die pharmakologisch verträglichen Alkali-, Erdalkali- oder Erdmetallsalze schwacher Säuren sowie die pharmakologisch verträglichen Hydroxide und Oxide von Erdalkali- und Erdmetallen genannt. Als beispielhaft hervorzuhebende Base sei Natriumcarbonat genannt.

Neben Füllstoff und Bindemittel kommen bei der Tablettenkernherstellung noch weitere Hilfsstoffe, insbesondere Gleit- und Trennmittel sowie Tabletten-Sprengmittel zum Einsatz.

Als Gleit- und Trennmittel seien beispielsweise Calciumsalze höherer Fettsäuren, wie z.B. Calciumstearat genannt.

Als Tabletten-Sprengmittel kommen insbesondere chemisch indifferente Mittel infrage. Als bevorzugtes Tabletten-Sprengmittel sei (quer)vernetztes Polyvinylpyrrolidon (z.B. Crospovidone) genannt.

Bezüglich der auf den Pellet- bzw. Tablettenkern aufzubringenden wasserlöslichen Zwischenschicht(en) wird auf solche wasserlöslichen Schichten verwiesen, wie sie üblicherweise vor der Aufbringung magensaftresistenter Schichten verwendet werden, oder wie sie z.B. in der DE-OS 39 01 151 beschrieben sind. Als für die Zwischenschicht verwendbare Filmpolymere seien beispielsweise Hydroxypropylmethylcellulose und/oder Polyvinylpyrrolidon genannt, denen gewünschtenfalls noch Weichmacher (wie etwa Propylenglykol) und/oder weitere Zusatz- und Hilfsstoffe (z.B. Puffer, Basen oder Pigmente) beigefügt werden können.

Welche magensaftresistenten äußeren Schichten verwendet werden können, ist dem Fachmann aufgrund seines Fachwissens bekannt. Vorteilhafterweise werden (zur Vermeidung organischer Lösungsmittel und da der erfindungsgemäße Kern nicht die aus dem Stand der Technik bekannte Wasserempfindlichkeit aufweist) wäßrige Dispersionen geeigneter magensaftresistenter Polymere, wie beispielsweise ein Methacrylsäure/Methacrylsäuremethylester-Copolymerisat, gewünschtenfalls unter Zusatz eines Weichmachers (z.B. Triethylacetat) verwendet.

Der Wirkstoff Pantoprazol ist bekannt aus dem europäischen Patent 166 287. Als Salze des Pantoprazols seien die im europäischen Patent 166 287 genannten Salze beispielhaft erwähnt. Ein bevorzugtes Salz ist das Natriumsalz.

Die Verwendung von Mannit als alleinigem Füllstoff für Tabletten erfordert ein geeignetes Bindemittel, das dem Kern eine ausreichende Härte verleihen muß. Bei dem für die Kern-Herstellung als Bindemittel verwendeten Polyvinylpyrrolidon handelt es sich insbesondere um ein Produkt mit höherem Molekulargewicht (ca. 300.000 bis 400.000). Als bevorzugtes Polyvinylpyrrolidon sei PVP 90 (Molekulargewicht ca. 360.000) genannt.

Die erfindungsgemäße orale Darreichungsform zeichnet sich gegenüber den aus dem Stand der Technik bekannten Darreichungsformen für andere $H^+/K^+$-ATPase-Hemmer mit Pyridylmethylsulfinyl-1H-benzimidazol-Struktur insbesondere dadurch aus, daß ein über 1,5 Gew.-% hinausgehender Wassergehalt im Tablettenkern nicht zu einer Verfärbung (Zersetzung) des Wirkstoffes führt. So werden auch bei einer höheren Restfeuchte im Granulat (von z.B. 5 bis 8 Gew.-%) stabile Tabletten erhalten.

Pellets können durch Auftragen einer Vorisolierung auf Saccharose-Starterpellets und anschließendes Auftragen einer 30 %igen isopropanolischen Wirkstofflösung mit Hydroxymethylpropylcellulose als Binder erhalten werden.

Der Auftrag der Isolierschicht kann analog zu den Tabletten auch unter Verwendung entsprechender Fertigdispersionen (z.B. Opadry) erfolgen. Der magensaftresistente Überzug erfolgt analog zu der Vorgehensweise bei Tabletten.

Weiterer Gegenstand der Erfindung ist ein Verfahren zur Herstellung der erfindungsgemäßen Arzneimittel in Pellet- oder Tablettenform, das dadurch gekennzeichnet ist, daß man den erfindungsgemäßen Kern herstellt, mit einer oder mehreren inerten wasserlöslichen Zwischenschichten umgibt und eine magensaftresistente äußere Schicht aufträgt.

Die folgenden Formulierungsbeispiele erläutern die Erfindung näher.

**Beispiele**

**1. Tabletten**

I. Tablettenkern

| a) Pantoprazol-Na-Sesquihydrat | 45,1 mg |
|---|---|
| b) Natriumcarbonat | 10,0 mg |
| c) Mannit | 42,7 mg |
| d) Crospovidone | 50,0 mg |
| e) PVP 90 (Povidone) | 4,0 mg |
| f) Calciumstearat | 3,2 mg |
| | 155,0 mg |

a) wird mit einem Teil von b), c) und d) vermischt. Der Rest von b) und c) wird in die klare wässrige Lösung von e) gegeben und mit b) auf einen pH-Wert > 10 eingestellt. Mit dieser Lösung wird in der Wirbelschicht granuliert. Dem getrockneten Granulat wird der Rest von d) sowie f) zugesetzt und das Granulat auf einer geeigneten Tablettenmaschine verpreßt.

II. Vorisolierung (Zwischenschicht)

| | |
|---|---|
| g) HPMC 2910, 3cps | 15,83 mg |
| h) PVP 25 | 0,32 mg |
| i) Titandioxid | 0,28 mg |
| j) LB Eisenoxid-gelb 100 E 172 | 0,025 mg |
| k) Propylenglykol | 3,54 mg |
| | 20,00 mg |
| Gesamtgewicht pro vorisoliertem Kern | 175,00 mg |

g) wird in Wasser gelöst und h) zugegeben und ebenfalls gelöst (A). i) und j) werden mit einem geeigneten Rührer in Wasser suspendiert (B). A und B werden vereinigt. Nach Zugabe von k) wird die Suspension unmittelbar vor der weiteren Verarbeitung gesiebt, bei der die unter I. erhaltenen Tablettenkerne in einem geeigneten Gerät mit der Suspension in ausreichender Schichtdicke überzogen werden.

III. Magensaftresistenter Überzug

| | |
|---|---|
| l) Eudragit® L 30 D | 13,64mg |
| m) Triethylcitrat | 1,36mg |
| | 15,00mg |
| Gesamtgewicht pro magensaftresistenter Filmtablette | 190,00mg |

l) wird mit Wasser verdünnt und m) zugesetzt. Die Dispersion wird vor der Verarbeitung gesiebt.

Auf die unter II. erhaltenen vorisolierten Kerne wird III. in geeigneten Apparaturen aufgesprüht.

**2. Pellets**

I. Starterpellets

| | |
|---|---|
| a) Saccharose Pellets (0,7-0,85 mm) | 950,0 g |
| b) Hydroxypropylmethylcellulose | 50,0 g |

a) wird mit der wäßrigen Lösung von b) in der Wirbelschicht (Wurster-Verfahren) besprüht.

II. Aktivpellets

| | |
|---|---|
| c) Pantoprazol-Na-Sesquihydrat | 403,0 g |
| d) Hydroxypropylmethylcellulose | 40,3 g |

c) und d) werden nacheinander in 30 % Isopropanol gelöst und auf 900 g der unter I. erhaltenen Starterpellets in der Wirbelschicht (Wurster-Verfahren) aufgesprüht.

III. Vorisolierung (Zwischenschicht)

Der Überzug erfolgt analog zu der bei den Tabletten beschriebenen Vorgehensweise im Kessel oder in der Wirbelschicht.

IV. Magensaftresistenter Überzug

Der Überzug erfolgt analog zu der bei den Tabletten beschriebenen Vorgehensweise im Kessel oder in der Wirbelschicht.
Anschließend werden die Pellets in Kapseln geeigneter Größe (z.B. 1) abgefüllt.

**Patentansprüche**

**Patentansprüche für folgende Vertragsstaaten : AT, BE, CH, DE, DK, FR, GB, IT LI, LU, MC, NL, PT, SE**

1. Oral zu applizierendes, mangensaftresistentes Arzneimittel in Pellet- oder Tablettenform, bei dem die Pellets bzw. Tabletten aus

   - einem Kern, in dem der Wirkstoff oder dessen physiologisch verträgliches Salz im Gemisch mit einem oder mehreren Bindemitteln, Füllstoffen und gewünschtenfalls anderen Tablettenhilfsstoffen und gewünschtenfalls einer oder mehreren basisch reagierenden physiologisch verträglichen anorganischen Verbindungen vorliegt,
   - einer oder mehreren diesen Kern umgebenden inerten, wasserlöslichen Zwischenschichten und
   - einer magensaftresistenen äußeren Schicht bestehen,

   dadurch gekennzeichnet, daß im Kern als Wirkstoff Pantoprazol, als Bindemittel Polyvinylpyrrolidon und/oder Hydroxypropylmethylcellulose und gewünschtenfalls als Füllstoff Mannit verwendet wird.

2. Arzneimittel nach Anspruch 1 in Tablettenform, dadurch gekennzeichnet, daß als Bindemittel Polyvinylpyrrolidon und/oder Hydroxypropylmethylcellulose und als Füllstoff Mannit verwendet wird.

3. Arzneimittel nach Anspruch 1 in Pelletform, dadurch gekennzeichnt, daß als Bindemittel Polyvinylpyrrolidon und/oder Hydroxypropylmethylcellulose verwendet wird.

4. Arzneimittel nach Anspruch 1 oder 2 oder 3, dadurch gekennzeichnet, daß als physiologisch verträgliches Wirkstoffsalz Pantoprazol-Natrium verwendet wird.

5. Arzneimittel nach Anspruch 1 oder 2 oder 3, dadurch gekennzeichnet, daß als basisch reagierende, physiologisch verträgliche anorganische Verbindungen pharmakologisch verträgliche Alkali-, Erdalkali- oder Erdmetallsalze schwacher Säuren oder pharmakologisch verträgliche Hydroxide oder Oxide von Erdalkali- oder Erdmetallen verwendet werden.

6. Arzneimittel nach Anspruch 1 oder 2 oder 3, dadurch gekennzeichnet, daß als basisch reagierende, physiologisch verträgliche anorganische Verbindung Natriumcarbonat verwendet wird.

7. Oral zu applizierendes, magensaftresistentes Arzneimittel in Pellet- oder Tablettenform, enthaltend im Pellet- bzw. Tablettenkern neben Pantoprazol oder einem Pantoprazol-Salz als Wirkstoff Polyvinylpyrrolidon und/oder Hydroxypropylmethylcellulose als Bindemittel und gewünschtenfalls Mannit als Füllstoff, unter Ausschluß der Bindemittel bzw. Füllstoffe Lactose, mikrokristalline Zellulose und Hydroxypropylzellulose.

**Patentansprüche für folgende Vertragsstaaten : ES, GR**

1. Verfahren zur Herstellung eines oral zu applizierenden, magensaftresistenten Arzneimittels in Pellet- oder Tablettenform, bei dem die Pellets bzw. Tabletten aus

- einem Kern, in dem der Wirkstoff oder dessen physiologisch verträgliches Salz im Gemisch mit einem oder mehreren Bindemitteln, Füllstoffen und gewünschtenfalls anderen Tablettenhilfsstoffen und gewünschtenfalls einer oder mehreren basisch reagierenden physiologisch verträglichen anorganischen Verbindungen vorliegt,
- einer oder mehreren diesen Kern umgebenden inerten, wasserlöslichen Zwischenschichten und
- einer magensaftresistenten äußeren Schicht bestehen,

dadurch gekennzeichnet, daß bei der Pellet- bzw. Tablettenherstellung im Kern als Wirkstoff Pantoprazol, als Bindemittel Polyvinylpyrrolidon und/oder Hydroxypropylmethylcellulose und gewünschtenfalls als Füllstoff Mannit verwendet wird.

2. Verfahren nach Anspruch 1 zur Herstellung eines Arzneimittels in Tablettenform, dadurch gekennzeichnet, daß als Bindemittel Polyvinylpyrrolidon und/oder Hydroxypropylmethylcellulose und als Füllstoff Mannit verwendet wird.

3. Verfahren nach Anspruch 1 zur Herstellung eines Arzneimittels in Pelletform, dadurch gekennzeichnet, daß als Bindemittel Polyvinylpyrrolidon und/oder Hydroxypropylmethylcellulose verwendet wird.

4. Verfahren nach Anspruch 1 oder 2 oder 3 zur Herstellung eines Arzneimittels, dadurch gekennzeichnet, daß als physiologisch verträgliches Wirkstoffsalz Pantoprazol-Natrium verwendet wird.

5. Verfahren nach Anspruch 1 oder 2 oder 3 zur Herstellung eines Arzneimittels, dadurch gekennzeichnet, daß als basisch reagierende, physiologisch verträgliche anorganische Verbindungen pharmakologisch verträgliche Alkali-, Erdalkali- oder Erdmetallsalze schwacher Säuren oder pharmakologisch verträgliche Hydroxide oder Oxide von Erdalkali- oder Erdmetallen verwendet werden.

6. Verfahren nach Anspruch 1 oder 2 oder 3 zur Herstellung eines Arzneimittels, dadurch gekennzeichnet, daß als basisch reagierende, physiologisch verträgliche anorganische Verbindung Natriumcarbonat verwendet wird.

7. Verfahren zur Herstellung eines oral zu applizierenden, magensaftresistenten Arzneimittels in Pellet- oder Tablettenform, dadurch gekennzeichnet, daß bei der Pellet- bzw. Tablettenkernherstellung neben Pantoprazol oder einem Pantoprazol-Salz als Wirkstoff Polyvinylpyrrolidon und/oder Hydroxypropylmethylcellulose als Bindemittel und gewünschtenfalls Mannit als Füllstoff verwendet wird, unter Ausschluß der Bindemittel bzw. Füllstoffe Lactose, mikrokristalline Zellulose und Hydroxypropylzellulose.

## Claims

**Claims for the following Contracting States : AT, BE, CH, DE, DK, FR, GB, IT, LI, LU, MC, NL, PT, SE**

1. Medicament in pellet or tablet form which is to be administered orally and is resistant to gastric juice, in which the pellets or tablets consist of

- a core in which the active compound or its physiologically tolerated salt is present as a mixture with one or more binders, fillers and if desired other tablet auxiliaries, and if desired one or more basic physiologically tolerated inorganic compounds,
- one or more inert, water-soluble intermediate layers surrounding this core and
- an outer layer which is resistant to gastric juice,

characterized in that, in the core, pantoprazole is used as the active compound, polyvinylpyrrolidone and/or hydroxypropylmethylcellulose is used as the binder and, if desired, mannitol is used as the filler.

2. Medicament according to Claim 1 in tablet form, characterized in that polyvinylpyrrolidone and/or hydroxypropylmethylcellulose is used as the binder and mannitol is used as the filler.

3. Medicament according to Claim 1 in pellet form, characterized in that polyvinylpyrrolidone and/or hydroxypropylmethylcellulose is used as the binder.

4. Medicament according to Claim 1 or 2 or 3, characterized in that pantoprazole-sodium is used as the physiologically tolerated active compound salt.

5. Medicament according to Claim 1 or 2 or 3, characterized in that pharmacologically tolerated alkali metal, alkaline earth metal or earth metal salts of weak acids or pharmacologically tolerated hydroxides or oxides of alkaline earth or earth metals are used as the basic, physiologically tolerated inorganic compounds.

6. Medicament according to Claim 1 or 2 or 3, characterized in that sodium carbonate is used as the basic, physiologically tolerated inorganic compound.

7. Medicament in pellet or tablet form which is to be administered orally and is resistant to gastric juice, containing in the pellet or tablet core besides pantoprazole or a pantoprazole salt as the active compound polyvinylpyrrolidone and/or hydroxypropylmethylcellulose as the binder and, if desired, mannitol as the filler, with the exception of the binders or fillers lactose, microcrystalline cellulose and hydroxypropylcellulose.

**Claims for the following Contracting States : ES, GR**

1. Process for the production of a medicament in pellet or tablet form which is to be administered orally and is resistant to gastric juice, in which the pellets or tablets consist of

   - a core in which the active compound or its physiologically tolerated salt is present as a mixture with one or more binders, fillers and if desired other tablet auxiliaries, and if desired one or more basic physiologically tolerated inorganic compounds,
   - one or more inert, water-soluble intermediate layers surrounding this core and
   - an outer layer which is resistant to gastric juice,

   characterized in that for the pellet or tablet production, in the core, pantoprazole is used as the active compound, polyvinylpyrrolidone and/or hydroxypropylmethylcellulose is used as the binder and, if desired, mannitol is used as the filler.

2. Process according to Claim 1 for the production of a medicament in tablet form, characterized in that polyvinylpyrrolidone and/or hydroxypropylmethylcellulose is used as the binder and mannitol is used as the filler.

3. Process according to Claim 1 for the production of a medicament in pellet form, characterized in that polyvinylpyrrolidone and/or hydroxypropylmethylcellulose is used as the binder.

4. Process according to Claim 1 or 2 or 3 for the production of a medicament, characterized in that pantoprazole-sodium is used as the physiologically tolerated active compound salt.

5. Process according to Claim 1 or 2 or 3 for the production of a medicament, characterized in that pharmacologically tolerated alkali metal, alkaline earth metal or earth metal salts of weak acids or pharmacologically tolerated hydroxides or oxides of alkaline earth or earth metals are used as the basic, physiologically tolerated inorganic compounds.

6. Process according to Claim 1 or 2 or 3 for the production of a medicament, characterized in that sodium carbonate is used as the basic, physiologically tolerated inorganic compound.

7. Process for the production of a medicament in pellet or tablet form which is to be administered orally and is resistant to gastric juice, characterized in that for the pellet or tablet core production besides pantoprazole or a pantoprazole salt as the active compound, polyvinylpyrrolidone and/or hydroxypropylmethylcellulose is used as the binder and, if desired, mannitol is used as the filler, with the exception of the binders or fillers lactose, microcrystalline cellulose and hydroxypropylcellulose.

**Revendications**

**Revendications pour les Etats contractants suivants : AT, BE, CH, DE, DK, FR, GB, IT, LI, LU, MC, NL, PT, SE**

1. Médicament résistant au suc gastrique, destiné à être administré par voie orale et se présentant sous forme de pilules ou de comprimés, lesquels pilules ou comprimés sont constitués

- d'un noyau dans lequel se trouve l'ingrédient actif ou l'un de ses sels physiologiquement compatibles, mélangé avec un ou plusieurs liants et charges, éventuellement avec d'autres adjuvants pour comprimés, et le cas échéant, avec un ou plusieurs composés minéraux physiologiquement compatibles à réaction basique,
- d'une ou plusieurs couches intermédiaires hydrosolubles et inertes, qui enrobent ce noyau, et
- d'une couche extérieure résistant au suc gastrique,

caractérisé en ce qu'on emploie, dans le noyau, du pantoprazol comme ingrédient actif, de la poly(vinyl-pyrrolidone) et/ou de l'hydroxy-propyl-méthyl-cellulose comme liant, et le cas échéant, du mannitol comme charge.

2. Médicament conforme à la revendication 1, sous forme de comprimés, caractérisé en ce qu'on emploie de la poly(vinyl-pyrrolidone) et/ou de l'hydroxypropyl-méthyl-cellulose comme liant, et du mannitol comme charge.

3. Médicament conforme à la revendication 1, sous forme de pilules, caractérisé en ce qu'on emploie de la poly(vinyl-pyrrolidone) et/ou de l'hydroxypropyl-méthyl-cellulose comme liant.

4. Médicament conforme à la revendication 1, 2 ou 3, caractérisé en ce qu'on emploie du sel sodique de pantoprazol comme sel physiologiquement compatible d'ingrédient actif.

5. Médicament conforme à la revendication 1, 2 ou 3, caractérisé en ce qu'on emploie, comme composés minéraux physiologiquement compatibles à réaction basique, des sels pharmacologiquement compatibles de métal alcalin, alcalino-terreux ou terreux et d'acide faible, ou des oxydes ou hydroxydes pharmacologiquement compatibles de métal alcalino-terreux ou terreux.

6. Médicament conforme à la revendication 1, 2 ou 3, caractérisé en ce qu'on emploie du carbonate de sodium comme compose minéral physiologiquement compatible à réaction basique,

7. Médicament résistant au suc gastrique, destiné à être administré par voie orale et se présentant sous forme de pilules ou de comprimés, lesquels pilules ou comprimés contiennent dans leur noyau, outre du pantoprazol ou un sel de pantoprazol comme ingrédient actif, de la poly(vinyl-pyrrolidone) et/ou de l'hydroxypropyl-méthyl-cellulose comme liant, et le cas échéant, du mannitol comme charge, à l'exclusion des liants et charges que sont le lactose, la cellulose microcristalline et l'hydroxypropyl-cellulose.

**Revendications pour les Etats contractants suivants : ES, GR**

1. Procédé de préparation d'un médicament résistant au suc gastrique, destiné à être administré par voie orale et se présentant sous forme de pilules ou de comprimés, lesquels pilules ou comprimés sont constitués

- d'un noyau dans lequel se trouve l'ingrédient actif ou l'un de ses sels physiologiquement compatibles, mélangé avec un ou plusieurs liants et charges, éventuellement avec d'autres adjuvants pour comprimés, et le cas échéant, avec un ou plusieurs composés minéraux physiologiquement compatibles à réaction basique,
- d'une ou plusieurs couches intermédiaires hydrosolubles et inertes, qui enrobent ce noyau, et
- d'une couche extérieure résistant au suc gastrique,

caractérisé en ce qu'on emploie, dans la préparation des pilules ou des comprimés, du pantoprazol comme ingrédient actif, de la poly(vinyl-pyrrolidone) et/ou de l'hydroxypropyl-méthyl-cellulose comme liant, et le cas échéant, du mannitol comme charge.

2. Procédé, conforme à la revendication 1, de préparation d'un médicament sous forme de comprimés, caractérisé en ce qu'on emploie de la poly(vinyl-pyrrolidone) et/ou de l'hydroxypropyl-méthyl-cellulose comme liant, et du mannitol comme charge.

3. Procédé, conforme à la revendication 1, de préparation d'un médicament sous forme de pilules, caractérisé en ce qu'on emploie emploie de la poly(vinyl-pyrrolidone) et/ou de l'hydroxypropyl-méthyl-cellulose comme liant.

4. Procédé, conforme à la revendication 1, 2 ou 3, de préparation d'un médicament, caractérisé en ce qu'on emploie du sel sodique de pantoprazol comme sel physiologiquement compatible d'ingrédient actif.

5. Procédé, conforme à la revendication 1, 2 ou 3, de préparation d'un médicament, caractérisé en ce qu'on emploie, comme composés minéraux physiologiquement compatibles à réaction basique, des sels pharmacologiquement compatibles de métal alcalin, alcalino-terreux ou terreux et d'acide faible, ou des oxydes ou hydroxydes pharmacologiquement compatibles de métal alcalino-terreux ou terreux.

6. Procédé, conforme à la revendication 1, 2 ou 3, de préparation d'un médicament, caractérisé en ce qu'on emploie du carbonate de sodium comme composé minéral physiologiquement compatible à réaction basique,

7. Procéédé de préparation d'un médicament résistant au suc gastrique, destiné à être administré par voie orale et se présentant sous forme de pilules ou de comprimés, caractérisé en ce qu'on emploie, dans la préparation de ces pilules ou comprimés, outre du pantoprazol ou un sel de pantoprazol comme ingrédient actif, de la poly(vinyl-pyrrolidone) et/ou de l'hydroxypropyl-méthyl-cellulose comme liant, et le cas échéant, du mannitol comme charge, à l'exclusion des liants et charges que sont le lactose, la cellulose microcristalline et l'hydroxy-propyl-cellulose.